# EUROPEAN PATENT SPECIFICATION

(11) **EP 2 095 815 B1**
(45) Date of publication and mention of the grant of the patent: **26.10.2011**
(21) Application number: 08380057.3
(22) Date of filing: 26.02.2008
(51) Int. Cl.: A61K 9/20, A61K 31/4365

(54) **Pharmaceutical formulations containing clopidogrel**
Pharmazeutische Formulierungen mit Clopidogrel
Formules pharmaceutiques contenant du clopidogrel

(43) Date of publication of application: 02.09.2009
(73) Proprietor: Laboratorios Lesvi, S.L., 08970 Sant Joan Despi (Barcelona) (ES)
(72) Inventor: Diez Martin, Ignacio, E-08980 Sant Feliu de Llobregat,Barcelona (ES); Ubeda Pérez, Carmen, E-08348 Cabrils Barcelona (ES); Ariño Ros, Maria Aranzazu, E-08028 Barcelona (ES)
(74) Representative: ABG Patentes, S.L.

(56) References cited:
- WO-A-2005/048992
- WO-A-2005/070464
- WO-A-2007/008045
- WO-A-2007/049868
- NDINDAYINO F ET AL: "Characterization and evaluation of isomalt performance in direct compression" INTERNATIONAL JOURNAL OF PHARMACEUTICS 19991028 NL, vol. 189, no. 1, 28 October 1999 (1999-10-28), pages 113-124, XP002486902 ISSN: 0378-5173
- DOERR T ET AL: "BEURTEILUNG DER AUFLOESEKINETIK ORALER PHARMAZEUTISCHER DARREICHUNGSFORMEN AUS VERSCHIEDENEN SACCHARIDEN UND ZUCHERAUSTAUSCHSTOFFEN" PHARMAZEUTISCHE INDUSTRIE, AULENDORF, DE, vol. 58, no. 10, 1 January 1996 (1996-01-01), pages 947-952, XP002050750 ISSN: 0031-711X

## Description

### FIELD OF THE INVENTION

The present invention relates to new stable pharmaceutical formulations for the oral administration of pharmaceutically acceptable salts of Clopidogrel. Specifically, it relates to oral pharmaceutical formulations in the form of a tablet containing a pharmaceutically acceptable salt of Clopidogrel and isomalt.

### BACKGROUND OF THE INVENTION

Clopidogrel is a known blood platelet aggregation inhibitor and antithrombotic pharmaceutical active ingredient. It is effective in treating and preventing various platelet-associated vascular diseases such as stroke, arteriosclerosis, myocardial infarction, angina pectoris, arrhythmia, peripheral arteries disease and Burger's disease. By inhibiting platelet aggregation, Clopidogrel reduces the chance of arterial blockage.

The chemical name of Clopidogrel is methyl (*S*)-alpha-(2-chlorophenyl)-6,7-dihydro-thieno[3,2-c]pyridine-5(4*H*)-acetate, with CAS Registry Number 113665-84-2 and structural formula I.

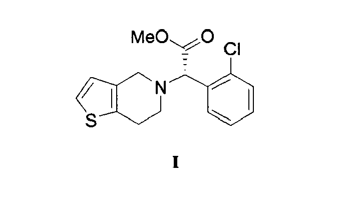

The CAS Registry Numbers of related salts are: 120202-65-5 for Clopidogrel hydrochloride, 120202-67-7 for Clopidogrel hydrobromide, 120202-66-6 for Clopidogrel hydrogensulphate, 744256-72-2 for Clopidogrel mesylate, 7744256-69-7 for Clopidogrel besylate.

The synthesis of Clopidogrel is disclosed, among other patents and patent applications, in EP0099802B1. Some salts of Clopidogrel are described in EP0281459A. EP1756116 describes different polymorphic forms (Forms A, B, C and D) of Clopidogrel hydrobromide and EP1087976 describes different polymorphs (Forms I and II) of Clopidogrel hydrogensulphate.

Clopidogrel is marketed as Plavix® and Iscover®. The products are in the form of tablets comprising an amount of the hydrogensulphate salt equivalent to 75 mg of the free base. The currently marketed tablets use hydrogenated castor oil, low substituted hydroxypropylcellulose, mannitol, microcrystalline cellulose and polyethylenglycol 6000 as excipients, and are coated. Earlier formulation registered in the regulatory offices used different excipients.

The desired dissolution profile shows at least 80 % dissolution at 30 minutes in 1000 mL of a pH 2.0 solution with paddles rotating at 50 rpm according to the dissolution described in the USP30 Clopidogrel monograph.

In EP0099802B1 it is described the use of lactose, icing or white sugar, rice or corn starch, alginic acid, magnesium stearate, arabic gum, shellac, glucose, white and carnauba wax, paraffin and new cochinealin to prepare tablets comprising Clopidogrel. EP0281459A describes the same excipients but their use is restricted to the dextrorotatory isomer of Clopidogrel and its salts. The hydrochloride, hydrogensulphate, hydrobromide and the taurocholate salts are explicitly disclosed in the application.

EP1161956A1 and WO2004026879 describe pharmaceutical formulation containing salts of Clopidogrel using a granulating process with specific excipients.

WO2005070464 describes a tablet formulation of Clopidogrel bisulphate where hydrogenated vegetable oil is used as lubricant and microcrystalline cellulose and lactose monohydrate as diluents.

EP 1310245 describes pharmaceutical tablet formulations comprising Clopidogrel bisulphate and a lubricant selected from zinc stearate, sodium stearyl fumarate and stearic acid. These tablets use methylcellulose as filler/binder. The document states that the tablets should preferably exclude microcrystalline cellulose which inhibits dissolution.

WO2007008045 describes Clopidogrel bisulphate formulations containing hydrogenated castor oil and sodium stearyl fumarate as lubricants and microcrystalline cellulose, starch and mannitol as diluents.

WO2007049868 describes a pharmaceutical formulation containing Clopidogrel hydrogensulphate using a specific ratio of starch and cellulose.

WO2007091279 describes Clopidogrel hydrogensulphate formulations using glycerol dibehenate as lubricant and anhydrous lactose and microcrystalline cellulose as diluents. This document also states that the use of microcrystalline cellulose in said formulations does not retard the disintegration and dissolution.

EP1847258 describes formulations containing Clopidogrel and a partially protected glycerine.

Clopidogrel is unstable, sensitive to moisture and to some excipients (*e*.*g*. magnesium stearate, as stated in EP1310245) and with low water solubility at a pH other than acid.

Furthermore, the inventors have found that some formulations described in the art, such as those containing sodium stearyl fumarate, result in high level of impurities in stability control conditions; in general, it has also been found that excipients that contain alkaline or alkaline earth metals salts tend to increase the impurities during storage as do excipients containing water or prone to absorb it.

The inventors have also found that even the commercial tablets of Clopidogrel hydrogensulphate (Plavix®) does not comply with the impurities and dissolution profile specifications when stored at 40°C and 75% humidity during six months.

Therefore, the formulation of pharmaceutically acceptable Clopidogrel salts challenges the formulation scientists to obtain tablets showing good chemical properties, i.e. tablets where Clopidogrel is not degraded and no impurities or degradation compounds are generated during storage, and that have good resistance to crushing and a desired dissolution profile.

Considering all this, there is still a need in the art to develop new oral formulations of pharmaceutically acceptable salts of Clopidogrel in the form of a tablet that allows good storage stability along with a desired dissolution profile.

### BRIEF DESCRIPTION OF THE INVENTION

The authors of the present invention have surprisingly found that the use of isomalt as a diluent allows obtaining stable pharmaceutical formulations of pharmaceutically acceptable salts of Clopidogrel in the form of a tablet, also showing a desirable dissolution profile. The examples provided show that the pharmaceutical formulations of the present invention are stable in terms of chemical properties and dissolution profile at 40°C at 75% relative humidity for at least 30 days. In fact, the dissolution profile of these formulations after 30 days showed over 80% dissolution at 30 minutes and good chemical properties. Therefore, the present invention relates to a composition in the form of a tablet which comprises isomalt and a pharmaceutically acceptable salt of Clopidogrel.

In a particular embodiment, the composition of the invention further comprises a disintegrant.

In another particular embodiment, the composition of the invention further comprises a lubricant.

In a particular embodiment, said tablet can be prepared by direct compression, wet granulation or dry granulation. Likewise, said tablet can be optionally film coated.

Finally, the present invention refers to a composition which comprises a pharmaceutically acceptable salt of clopidogrel and sucrose stearate.

### DETAILED DESCRIPTION OF THE INVENTION

The present invention describes a composition in the form of a tablet comprising a pharmaceutically acceptable salt of Clopidogrel and isomalt as a diluent. The use of isomalt as a diluent unexpectedly allows to obtain stable pharmaceutical formulations comprising pharmaceutically acceptable salts of Clopidogrel which also exhibit a desirable dissolution profile.

By the term 'composition' it is understood a physical combination of the components including a pharmaceutically acceptable salt of Clopidogrel and isomalt.

By the term 'pharmaceutically acceptable salt of Clopidogrel' it is understood any acid addition salt of acids that are physiologically tolerable and do not typically produce an allergic or similar untoward reaction, such as gastric upset, dizziness and the like, when administered to a human. Preferably, as used herein, the term "pharmaceutically acceptable" means acids approved by a regulatory agency worldwide or listed in the European Pharmacopoeia, the U.S. Pharmacopoeia or other generally recognized pharmacopoeia for use in animals, and more particularly in humans. Said acid can be chosen from mineral acid addition salts such as hydrochloride, hydrobromide, hydroiodide, sulphate, hydrogensulphate, nitrate, phosphate, and organic acid addition salts such as mesylate, besylate, acetate, maleate, fumarate, citrate, oxalate, succinate, tartrate, malate, mandelate, methanesulfonate and p-toluenesulfonate.

The preparation of the pharmaceutically acceptable salts of Clopidogrel can be carried out by methods known in the art. For instance, they are synthesized by reacting Clopidogrel with a stoichiometric amount of the appropriate acid in water or in an organic solvent or in a mixture of the two. Generally, non-aqueous media like ether, ethyl acetate, ethanol, isopropanol or acetonitrile are preferred.

In a particular embodiment of the invention, the pharmaceutically acceptable salt of Clopidogrel is selected from the group consisting of Clopidogrel hydrogensulphate, Clopidogrel hydrobromide, Clopidogrel hydrochloride, Clopidogrel besylate and Clopidogrel mesylate. In a preferred embodiment, the pharmaceutically acceptable salt of Clopidogrel is Clopidogrel hydrobromide. In another preferred embodiment, the pharmaceutically acceptable salt of Clopidogrel is Clopidogrel hydrogensulphate. Even in another preferred embodiment, the pharmaceutically acceptable salt of Clopidogrel is Clopidogrel besylate.

In another particular embodiment, the pharmaceutically acceptable salt of Clopidogrel may be present in a polymorphic form. It is intended that the invention encompasses all such forms. In a preferred embodiment, the pharmaceutically acceptable salt of Clopidogrel is Clopidogrel hydrobromide Form A. This crystalline form is as described in EP 1756116, In another preferred embodiment, the pharmaceutically acceptable salt of Clopidogrel is Clopidogrel hydrogensulphate Form 1, this crystalline form is as described in EP1087976,

On the other hand, the term 'Isomalt' is defined according to the European Pharmacopeia 6.0, where it is described as a mixture of 6-*O*-α-D-glucopyranosyl-D-sorbitol (1,6-GPS) and 1-*O*-α-D-glucopyranosyl-D-mannitol dihydrate (1,1-GPM·2H₂O), in different weight ratios. However, although said ratios are variable, neither of the two components is less than 3% by weight.

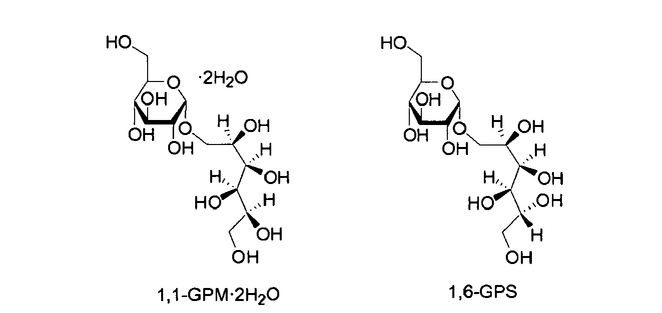

In a preferred embodiment of the invention, Isomalt is selected to have a weight ratio of 1,1-GPM·2H₂O to 1,6-GPS comprised between 1:6 and 4:1, more preferably between 1:4 and 2:1. In one particular embodiment said ratio is comprised between 1:2.8 and 1:3.2 and in another particular embodiment is comprised between 1:0.8 and 1:1.2.

Isomalt is a sugar replacer derived exclusively from sucrose. Isomalt is available in a wide range of particle sizes, from granulate to powder.

In a particular embodiment, the isomalt is an agglomerated isomalt understanding as such an isomalt in granulate form with, preferably, the following particle size distribution: d₁₀=50-150 µm, d₅₀=200-400 µm and d₉₀=300-600 µm. The terms d₁₀, d₅₀ and d₉₀ refer to the diameter of the granules, being the subscript the percentage of granules that present the specified diameter.

In another particular embodiment, the isomalt is a powder isomalt with the following particle size distribution: d₁₀=2-15 µm, d₅₀=18-35 µm and d₉₀=38-100 µm. This powder isomalt can be obtained by milling and sieving the agglomerated isomalt defined above to obtain the desired particle size distribution.

The agglomerated and powdered forms of isomalt used in the present invention can be those commercially available under the trademark GalenIQ720, 721, 800 and 810.

The use of other sugars, such as hydrated lactose, resulted in formulations without the desired properties.

In a particular embodiment of the invention, the amount of isomalt is comprised between 30 and 65% by weight with respect to the total weight of the composition. Preferably, this amount is comprised between 39 and 55% by weight.

In a particular embodiment, the composition of the present invention further comprises a lubricant. In a preferred embodiment, said lubricant is selected from one or more of the following: glycerol dibehenate; hydrogenated vegetable oils, such as hydrogenated castor oil, hydrogenated cottonseed oil; glycerol mono/di/tri palmitate/stearate esters; macrogol 3000-6000; stearic acid; poloxamers and sucrose fatty acid esters, such as sucrose stearate. More preferably, the lubricant is sucrose stearate since this compound renders the formulation with good chemical and physical properties.

Optionally, the lubricant can be mixed with talc.

The glycerol mono/di/tri palmitate/stearate esters are glycerine esters with one, two or three palmitate acid or stearate acid or with mixtures thereof.

Preferably, the amount of lubricant is comprised between 0.5 and 7% by weight with respect to the total weight of the composition. More preferably, said amount of lubricant is comprised between 1 and 5% by weight.

In another particular embodiment, the composition of the present invention further comprises a disintegrant. In a preferred embodiment, said disintegrant is selected from one or more of the following: crospovidone, hydroxypropyl cellulose and different kinds of starch, such as corn starch, potato starch or partial alpha starch.

Preferably, the amount of disintegrant is comprised between 1 and 20% by weight with respect to the total weight of the composition. More preferably, said amount of disintegrant is comprised between 2 and 15% by weight.

In another particular embodiment, the composition of the invention has the following quantitative formulation:

| **Compound** | **Percentage (% by weight)** |
|---|---|
| **Pharmaceutically acceptable salt of Clopidogrel** | 25-50 % |
| **Isomalt** | 30-65 % |
| **Disintegrant** | 1-20 % |
| **Lubricant** | 0.5-7 % |

The tablets of the present invention can be obtained by procedures which are well-known in the art.

In a particular embodiment, the tablet of the invention is prepared by direct compression. This procedure may be carried out by uniformly mixing together all the components in powder form, except the lubricant, and once a homogeneous powdered mixture is obtained, adding the lubricant thereto. The resulting mixture is then subjected to compression to provide a solid preparation in the form of a tablet. The compression can be carried out by any known method. For example, the mixture is fed to a die of a tablet press and sufficient pressure is applied to form the solid tablet. Such pressure can vary, and typically ranges about 1-30 KN, being particularly preferable 3-15 KN. Direct compression is the easiest way of manufacturing tablets and has the great advantage of having a low manufacturing cost. Moreover, it uses conventional equipment and a limited number of process steps.

In another particular embodiment, the tablet of the invention can be prepared by wet granulation. In this process, the pharmaceutically acceptable salt of Clopidogrel, part of the isomalt and, optionally, part of the disintegrant are granulated with water, alcohol or a mixture thereof or with a binder solution in order to obtain a solid preparation in the form of granules. The resulting granules are then sized to the desired particle size. The rest of the excipients, except the lubricant, are blended and mixed with the resulting granules and finally, the lubricant is added thereto. The resulting mixture is then compressed by a method such as the one described above. In a particular embodiment, the isomalt used in the wet granulation process is an agglomerated isomalt with the following particle size distribution: d₁₀=50-150 µm, d₅₀=200-400 µm and d₉₀=300-600 µm. In another particular embodiment the isomalt is milled with d₁₀=2-15 µm, d₅₀=18-35 µm and d₉₀=38-100 µm.

The binder solution used in the wet granulation process includes, for example, a solution of a binder such as hydroxypropyl methyl cellulose, carboxyvinyl polymer, alpha starch, polyvinylpyrrolidone, gum Arabic, gelatin, pullulan or the like.

In another particular embodiment, the tablet of the invention can be prepared by dry granulation. In this process, the pharmaceutically acceptable salt of Clopidogrel is mixed with part of all excipients and the resulting mixture is compacted and milled into the desired size prior to tabletting. The granules obtained are, then, mixed with the rest of the excipients and, finally, compressed.

Both granulation methods include, for example, rolling granulation method, fluidized-bed granulation, high shear or low shear granulation and the like.

The resulting compressed solid formulations possess a suitable strength and hardness and do not disintegrate during distribution and storage.

The tablets of pharmaceutically acceptable salt of Clopidogrel of the present invention are optionally film coated. The film coating can be chosen from the coatings available to the skilled in the art without special requirements, such as hydroxypropylmethyl cellulose or polyvinyl alcohol.

As it has been stated above, Clopidogrel is a known blood platelet aggregation inhibitory and antithrombotic pharmaceutical active ingredient. By inhibiting platelet aggregation, Clopidogrel reduces the chance of arterial blockage. Therefore, the oral pharmaceutical formulations of the invention are useful in treating and preventing various platelet-associated vascular diseases such as stroke, arteriosclerosis, myocardial infarction, angina pectoris, arrhythmia, peripheral arteries disease and Burger's disease.

In the following, the present invention is further illustrated by examples. They in the claims.

### EXAMPLES

### Example 1:

### Quantitative formulation:

| **Role** | **Compound** | **Per tablet (mg)** | **(%)** |
|---|---|---|---|
| **API** | Clopidogrel hydrobromide | 94.00 * | 37.60 |
| **Diluent** | Isomalt (GPM:GPS 1:3) | 117.50 | 47.00 |
| **Disintegrant** | Pregelatinized starch | 26.00 | 10.40 |
| | Glycerol dibehenate | 9.30 | 3.72 |
| **Lubricants** | Hydrogenated castor oil | 3.20 | 1.28 |
| | TOTAL | 250.00 | |

| | | | |
|---|---|---|---|
| * equivalent to 75.1 mg of Clopidogrel free base. | | | |

The used isomalt was in agglomerated form.

All the components, except the lubricants were blended and mixed. Then the lubricants were added and blended, the resulting mixture is tabletted by direct compression. Optionally the tablets can be film coated. The resulting tablets were homogeneous, with good resistance to crushing, a desired dissolution profile and good chemical properties.

Stability studies were performed on the resulting tablets packaged on Aluminium/aluminium and aluminium/PVDC blisters at 40°C at 75% RH for 30 days. The dissolution profile of these tablets after 30 days showed over 80% dissolution at 30 minutes and good chemical properties.

When other sugars, such as hydrated lactose, or sugar alcohols, such as sorbitol, were used instead of isomalt, the desired properties were not obtained.

### Example 2:

### Quantitative formulation:

| **Role** | **Compound** | **Per tablet (mg)** | **(%)** |
|---|---|---|---|
| **API** | Clopidogrel hydrogensulphate | 98.00 * | 39.20 |
| **diluent** | Isomalt (GPM:GPS 1:3) | 119.50 | 47.80 |
| **disintegrant** | Hydroxypropyl cellulose | 25.00 | 10.00 |
| **lubricant** | Sucrose stearate | 7.50 | 3.00 |
| | TOTAL | 250.00 | |

| | | | |
|---|---|---|---|
| * equivalent to 75.1 mg of Clopidogrel free base. | | | |

The used isomalt was in agglomerated form.

Following the same methodology of preparation as described in example 1, homogeneous tablets were obtained showing good resistance to crushing, desired dissolution profile and good chemical properties. Optionally, the tablets can be film coated.

Stability studies were performed on the resulting tablets packaged on Aluminium/aluminium and aluminium/PVDC blisters at 40°C at 75% RH for 30 days. The dissolution profile of these tablets after 30 days showed over 80% dissolution at 30 minutes and good chemical properties.

### Example 3:

### Quantitative formulation:

| **Role** | **Compound** | **Per tablet (mg)** | **(%)** |
|---|---|---|---|
| **API** | Clopidogrel hydrogensulphate | 98.00 * | 39.20 |
| **Diluent** | Isomalt (GPM:GPS 1:3) | 113.50 | 45.40 |
| **Disintegrant** | Hydroxypropyl cellulose | 26.00 | 10.40 |
| | Glycerol dibehenate | 9.30 | 3.72 |
| **Lubricants** | Hydrogenated castor oil | 3.20 | 1.28 |
| | TOTAL | 250.00 | |

| | | | |
|---|---|---|---|
| * equivalent to 75.1 mg of Clopidogrel free base. | | | |

The used isomalt was in milled form.

Following the same methodology of preparation as described in example 1 homogeneous tablets, with good resistance to crushing, desired dissolution profile and good chemical properties were obtained. Optionally the tablets can be film coated.

Stability studies were performed on the resulting tablets packaged on Aluminium/aluminium and aluminium/PVDC blisters at 40°C at 75% RH for 30 days. The dissolution profile of these tablets after 30 days showed over 80% dissolution at 30 minutes and good chemical properties.

### Example 4:

Using the same quantitative formulation as in examples 1, 2 or 3 the Clopidogrel salt and half of the isomalt were mixed and wet granulated (with water). The resulting granules were mixed with the remaining isomalt and the disintegrant. Finally the lubricants were added and mixed. The obtained mixture was tabletted. The resulting tablets were homogeneous, with good resistance to crushing and desired dissolution profile. Optionally the tablets can be film coated.

Stability studies were performed on the resulting tablets packaged on Aluminium/aluminium and aluminium/PVDC blisters at 40°C at 75% RH for 30 days. The dissolution profile of these tablets after 30 days showed over 80% dissolution at 30 minutes and good chemical properties.

Similar results were obtained using a 1:1 mixture of ethanol and isopropanol during the wet granulation process.

### Example 5:

Using the same quantitative formulation as in example 1 the Clopidogrel hydrobromide, the isomalt and half of the disintegrant and half of the lubricants were blended, mixed and compacted. The obtained tablets were dry granulated, and the resulting granules were mixed with the remaining lubricant and the remaining disintegrant and tabletted. The resulting tablets were homogeneous, with good resistance to crushing and the desired dissolution profile. Optionally the tablets can be film coated.

Stability studies were performed on the resulting tablets packaged on Aluminium/aluminium and aluminium/PVDC blisters at 40°C at 75% RH for 30 days. The dissolution profile of these tablets after 30 days showed over 80% dissolution at 30 minutes and good chemical properties.

Similar results were obtained using this preparation methodology and the quantitative formulations described in the examples 2 and 3.

### Example 6:

### Quantitative formulation:

| **Role** | **Compound** | **Per tablet (mg)** | **(%)** |
|---|---|---|---|
| **API** | Clopidogrel hydrogensulphate | 98.00 * | 39.20 |
| **Diluent** | Isomalt (GPM:GPS 1:3) | 108.50 | 43.40 |
| **Disintegrants** | Hydroxypropyl cellulose | 26.00 | 10.40 |
| | Crospovidone | 5.00 | 2.00 |
| **Lubricant** | Glycerol dibehenate | 12.50 | 5.00 |
| | TOTAL | 250.00 | |

| | | | |
|---|---|---|---|
| * equivalent to 75.1 mg of Clopidogrel free base. | | | |

The used isomalt was in agglomerated form.

Following the same methodology of preparation as described in example 4 homogeneous tablets, with good resistance to crushing, desired dissolution profile and good chemical properties were obtained. Optionally the tablets can be film coated.

Stability studies were performed on the resulting tablets packaged on Aluminium/aluminium and aluminium/PVDC blisters at 40°C at 75% RH for 30 days. The dissolution profile of these tablets after 30 days showed over 80% dissolution at 30 minutes and good chemical properties.

Instead of glycerol dibehenate the following lubricants were used obtaining similar results: hydrogenated castor oil, macrogol 3000-6000, stearic acid, poloxamer 188 and sucrose stearate.

### Example 7:

### Quantitative formulation:

| **Role** | **Compound** | **Per tablet (mg)** | **(%)** |
|---|---|---|---|
| **API** | Clopidogrel besylate | 111.90 * | 44.76 |
| **Diluent** | Isomalt (GPM:GPS 1:1) | 105.60 | 42.24 |
| **Disintegrant** | Hydroxypropyl cellulose | 25.00 | 10.00 |
| **Lubricant** | Glycerol dibehenate | 7.50 | 3.00 |
| | TOTAL | 250.00 | |

| | | | |
|---|---|---|---|
| * equivalent to 75.0 mg of Clopidogrel free base. | | | |

The used isomalt was in agglomerated form.

Following the same methodology of preparation as described in example 1, 4 or 5 homogeneous tablets, with good resistance to crushing, desired dissolution profile and good chemical properties were obtained. Optionally the tablets can be film coated.

Stability studies were performed on the resulting tablets packaged on Aluminium/aluminium and aluminium/PVDC blisters at 40°C at 75% RH for 30 days. The dissolution profile of these tablets after 30 days showed over 80% dissolution at 30 minutes and good chemical properties.

### Example 8:

Using the same quantitative formulation as in examples 1, 2 or 3 the Clopidogrel salt, half of the isomalt (GPM:GPS 1:1 in agglomerted form) and half of the disintegrant were mixed and wet granulated (with water). The resulting granules were mixed with the remaining isomalt and disintegrant. Finally the lubricants were added and mixed. The obtained mixture was tabletted. The resulting tablets were homogeneous, with good resistance to crushing and desired dissolution profile. Optionally the tablets can be film coated.

Stability studies were performed on the resulting tablets packaged on Aluminium/aluminium and aluminium/PVDC blisters at 40°C at 75% RH for 30 days. The dissolution profile of these tablets after 30 days showed over 80% dissolution at 30 minutes and good chemical properties.

Similar results were obtained using a 1:1 mixture of ethanol and isopropanol during the wet granulation process.

## Claims

1. A composition in the form of a tablet which comprises a pharmaceutically acceptable salt of Clopidogrel and isomalt.

2. A composition according to claim 1 wherein the pharmaceutically acceptable salt of Clopidogrel is selected from the group consisting of Clopidogrel hydrogensulphate, Clopidogrel hydrobromide, Clopidogrel hydrochloride, Clopidogrel besylate and Clopidogrel mesylate.

3. A composition according to claim 1 or 2, wherein the pharmaceutically acceptable salt of Clopidogrel is Clopidogrel hydrobromide.

4. A composition according to claim 1 or 2, wherein the pharmaceutically acceptable salt of Clopidogrel is Clopidogrel hydrogensulphate.

5. A composition according to claim 1 or 2, wherein the pharmaceutically acceptable salt of Clopidogrel is Clopidogrel besylate.

6. A composition according to claim 3, wherein the Clopidogrel hydrobromide is Clopidogrel hydrobromide Form A.

7. A composition according to claim 4, wherein the Clopidogrel hydrogensulphate is Clopidogrel hydrogensulphate Form 1.

8. A composition according to anyone of claims 1 to 7, wherein the amount of isomalt is comprised between 30 and 65% by weight, more preferably between 39 and 50% by weight, with respect to the total weight of the composition.

9. A composition according to anyone of claims 1 to 8 wherein isomalt is selected to have a weight ratio of 1,1-GPM·2H2O to 1,6-GPS comprised between 1:6 and 4:1, more preferably between 1:4 and 2:1.

10. A composition according to anyone of claims 1 to 9 which further comprises a lubricant, optionally mixed with talc.

11. A composition according to claim 10 wherein the lubricant is selected from one or more of the following: glycerol dibehenate, hydrogenated vegetable oils, glycerol mono/di/tri palmitate/stearate esters, macrogol 3000-6000, stearic acid, poloxamers and sucrose fatty acid esters.

12. A composition according to claim 11 wherein the lubricant is sucrose stearate.

13. A composition according to anyone of claims 10 to 12 wherein the amount of the lubricant is 0.5-7% by weight, more preferably is 1-5% by weight, with respect to the total weight of the composition.

14. A composition according to anyone of claims 1 to 13 which further comprises a disintegrant.

15. A composition according to claim 14 wherein the disintegrant is selected from one or more of the following: crospovidone, hydroxypropyl cellulose and other kind of starch selected from corn starch, potato starch, partial alpha starch and hydroxypropyl starch.

16. A composition according to claim 14 or 15 wherein the amount of the disintegrant is 1 to 20% by weight, more preferably is 2 to 15% by weight with respect to the total weight of the composition.

17. A composition according to anyone of the preceding claims having the following quantitative formulation:
- pharmaceutically acceptable salt of Clopidogrel: 25-50% by weight
- isomalt: 30-65% by weight
- disintegrant: 1-20% by weight
- lubricant: 0.5-7% by weight.

18. A composition according to any preceding claim which is further film coated.

19. A process for the preparation of a composition as defined in claims 1 to 17 by direct compression, wet granulation or dry granulation of its components.

## Patentansprüche

1. Zusammensetzung in der Form einer Tablette, die ein pharmazeutisch verträgliches Salz von Clopidogrel und Isomalt umfasst.

2. Zusammensetzung nach Anspruch 1, wobei das pharmazeutisch verträgliche Salz von Clopidogrel ausgewählt ist aus der Gruppe, bestehend aus Clopidogrel-Hydrogensulfat, Clopidogrel-Hydrobromid, Clopidogrel-Hydrochlorid, Clopidogrel-Besylat und Clopidogrel-Mesylat.

3. Zusammensetzung nach Anspruch 1 oder 2, wobei das pharmazeutisch verträgliche Salz von Clopidogrel Clopidogrel-Hydrobromid ist.

4. Zusammensetzung nach Anspruch 1 oder 2, wobei das pharmazeutisch verträgliche Salz von Clopidogrel Clopidogrel-Hydrogensulfat ist.

5. Zusammensetzung nach Anspruch 1 oder 2, wobei das pharmazeutisch verträgliche Salz von Clopidogrel Clopidogrel-Besylat ist.

6. Zusammensetzung nach Anspruch 3, wobei das Clopidogrel-Hydrobromid Clopidogrel-Hydrobromid Form A ist.

7. Zusammensetzung nach Anspruch 4, wobei das Clopidogrel-Hydrogensulfat Clopidogrel-Hydrogensulfat Form 1 ist.

8. Zusammensetzung nach einem der Ansprüche 1 bis 7, wobei die Menge Isomalt zwischen 30 und 65 Gew.-%, bevorzugter zwischen 39 und 50 Gew.-%, bezogen auf das Gesamtgewicht der Zusammensetzung, liegt.

9. Zusammensetzung nach einem der Ansprüche 1 bis 8, wobei Isomalt so ausgewählt ist, dass es ein Gewichtsverhältnis von 1,1-GPM·2H₂O zu 1,6-GPS aufweist, das zwischen 1:6 und 4: 1, bevorzugter zwischen 1:4 und 2:1 liegt.

10. Zusammensetzung nach einem der Ansprüche 1 bis 9, die weiter ein Gleitmittel, gegebenenfalls vermischt mit Talkum, umfasst.

11. Zusammensetzung nach Anspruch 10, wobei das Gleitmittel ausgewählt ist aus einem oder mehreren der folgenden: Glyceroldibehenat, hydrierten Pflanzenölen, Glycerolmono/di/tripalmitat/stearatestern, Macrogol 3000-6000, Stearinsäure, Poloxameren und Saccharosefettsäureestern.

12. Zusammensetzung nach Anspruch 11, wobei das Gleitmittel Saccharosestearat ist.

13. Zusammensetzung nach einem der Ansprüche 10 bis 12, wobei die Menge des Gleitmittels 0,5-7 Ges.-%, bevorzugter 1-5 Gew.-%, bezogen auf das Gesamtgewicht der Zusammensetzung, beträgt.

14. Zusammensetzung nach einem der Ansprüche 1 bis 13, die weiter ein Desintegrationsmittel umfasst.

15. Zusammensetzung nach Anspruch 14, wobei das Desintegrationsmittel ausgewählt ist aus einem oder mehreren der Folgenden: Crospovidon, Hydroxypropylcellulose und anderen Stärkearten, ausgewählt aus Maisstärke, Kartoffelstärke, partieller Alphastärke und Hydroxypropylstärke.

16. Zusammensetzung nach Anspruch 14 oder 15, wobei die Menge des Desintegrationsmittels 1 bis 20 Gew.-%, bevorzugter 2 bis 15 Gew.-%, bezogen auf das Gesamtgewicht der Zusammensetzung, beträgt.

17. Zusammensetzung nach einem der vorangehenden Ansprüche, mit der folgenden quantitativen Formulierung:
- pharmazeutisch verträgliches Salz von Clopidogrel: 25-50 Gew.-%
- Isomalt: 30-65 Gew.-%
- Desintegrationsmittel: 1-20 Gew.-%
- Gleitmittel: 0,5-7 Gew.-%.

18. Zusammensetzung nach einem vorangehenden Ansprüche, die weiter filmbeschichtet ist.

19. Verfahren zur Herstellung einer Zusammensetzung, wie definiert in den Ansprüchen 1 bis 17, durch Direktverpressung, Nassgranulation oder Trockengranulation ihrer Bestandteile.

## Revendications

1. Une composition sous la forme d'un comprimé qui comprend un sel pharmaceutiquement acceptable de clopidogrel et d'isomalt.

2. Une composition selon la revendication 1, dans laquelle le sel pharmaceutiquement acceptable de clopidogrel est sélectionné dans le groupe constitué par l'hydrogénosulfate de clopidogrel, le bromhydrate de clopidogrel, le chlorhydrate de clopidogrel, le bésylate de clopidogrel et le mésylate de clopidogrel.

3. Une composition selon la revendication 1 ou 2, dans laquelle le sel pharmaceutiquement acceptable de clopidogrel est le bromhydrate de clopidogrel.

4. Une composition selon la revendication 1 ou 2, dans laquelle le sel pharmaceutiquement acceptable de clopidogrel est l'hydrogénosulfate de clopidogrel.

5. Une composition selon la revendication 1 ou 2, dans laquelle le sel pharmaceutiquement acceptable de clopidogrel est le bésylate de clopidogrel.

6. Une composition selon la revendication 3, dans laquelle le bromhydrate de clopidogrel est le bromhydrate de clopidogrel Forme A.

7. Une composition selon la revendication 4, dans laquelle l'hydrogénosulfate de clopidogrel est l'hydrogénosulfate de clopidogrel Forme 1.

8. Une composition selon l'une quelconque des revendications 1 à 7, dans laquelle la quantité d'isomalt est comprise entre 30 et 65 % en poids, plus préférablement entre 39 et 50 % en poids, par rapport au poids total de la composition.

9. Une composition selon l'une quelconque des revendications 1 à 8, dans laquelle l'isomalt est sélectionné pour avoir un rapport pondéral du 1,1-GPM·2H₂O sur le 1,6-GPS compris entre 1:6 et 4:1, plus préférablement entre 1:4 et 2:1.

10. Une composition selon l'une quelconque des revendications 1 à 9 qui comprend en outre un lubrifiant, facultativement mélangé avec du talc.

11. Une composition selon la revendication 10, dans laquelle le lubrifiant est sélectionné parmi un ou plusieurs des éléments suivants : dibéhénate de glycérol, huiles végétales hydrogénées, esters de mono/di/tri palmitate/stéarate de glycérol, macrogol 3000-6000, acide stéarique, poloxamères et esters d'acides gras de saccharose.

12. Une composition selon la revendication 11, dans laquelle le lubrifiant est le stéarate de saccharose.

13. Une composition selon l'une quelconque des revendications 10 à 12, dans laquelle la quantité du lubrifiant est de 0,5 à 7 % en poids, plus préférablement de 1 à 5 % en poids, par rapport au poids total de la composition.

14. Une composition selon l'une quelconque des revendications 1 à 13 qui comprend en outre un délitant.

15. Une composition selon la revendication 14, dans laquelle le délitant est sélectionné parmi un ou plusieurs des éléments suivants : crospovidone, hydroxypropylcellulose et autre type d'amidon sélectionné parmi l'amidon de maïs, l'amidon de pomme de terre, l'alpha-amidon partiel et l'hydroxypropylamidon.

16. Une composition selon la revendication 14 ou 15, dans laquelle la quantité du délitant est de 1 à 20 % en poids, est plus préférablement de 2 à 15 % en poids par rapport au poids total de la composition.

17. Une composition selon l'une quelconque des revendications précédentes ayant la formulation quantitative suivante :
- sel pharmaceutiquement acceptable de clopidogrel : 25 à 50 % en poids
- isomalt : 30 à 65 % en poids
- délitant : 1 à 20 % en poids
- lubrifiant : 0,5 à 7 % en poids.

18. Une composition selon l'une quelconque des revendications précédentes qui est en outre revêtue d'un film.

19. Un procédé pour la préparation d'une composition telle qu'elle est définie dans les revendications 1 à 17 par compression directe, granulation humide ou granulation à sec de ses composants.
